**Europäisches Patentamt**

**European Patent Office**

**Office européen des brevets**

(19)

(11) Veröffentlichungsnummer: **0 355 176 B1**

(12)

# EUROPÄISCHE PATENTSCHRIFT

(45) Veröffentlichungstag der Patentschrift: **16.12.92**

(51) Int. Cl.⁵: **A61B 17/22**, G10K 11/30

(21) Anmeldenummer: **88113361.5**

(22) Anmeldetag: **17.08.88**

(54) **Vorrichtung zum berührungslosen Zertrümmern eines Konkrementes.**

(43) Veröffentlichungstag der Anmeldung:
**28.02.90 Patentblatt 90/09**

(45) Bekanntmachung des Hinweises auf die
Patenterteilung:
**16.12.92 Patentblatt 92/51**

(84) Benannte Vertragsstaaten:
**DE FR GB NL**

(56) Entgegenhaltungen:
**EP-A- 0 254 104**
**DE-A- 2 940 658**
**US-A- 3 913 061**

(73) Patentinhaber: **SIEMENS AKTIENGESELL-
SCHAFT**
**Wittelsbacherplatz 2**
**W-8000 München 2(DE)**

(72) Erfinder: **Hassler, Dietrich, Dipl.-Ing.**
**Flurweg 3**
**W-8525 Uttenreuth(DE)**
Erfinder: **Schmidt, Erhard, Ing.-grad.**
**Heuwaagstrasse 20**
**W-8520 Erlangen(DE)**
Erfinder: **Reichenberger, Helmut, Dr. rer. nat.**
**Begonienstrasse 28**
**W-8501 Eckental(DE)**

Rank Xerox (UK) Business Services

## Beschreibung

Die Erfindung betrifft eine Vorrichtung zum berührungslosen Zertrümmern eines Konkrementes im Körper eines Lebewesens mit einer Stoßwellenquelle zum Erzeugen einer Stoßwelle in einem die Stoßwelle übertragenden Medium, wobei die Stoßwelle auf einen Bereich im Körper des Lebewesens fokussiert ist.

Vorrichtungen dieser Art werden in der Medizin beispielsweise zur Zerstörung von Gallen- und Nierensteinen eingesetzt. Der Einsatz dieser Vorrichtungen ist sehr vorteilhaft, da hierdurch ein operativer Eingriff bzw. das Heranführen von Sonden und Geräten zur Entfernung der Konkremente mit einer Gefährdung des Patienten durch Infektion und/oder Verletzung entfällt.

Eine Vorrichtung der eingangs genannten Art ist beispielsweise in der DE-OS 33 28 039 beschrieben. Die von einer Stoßwellenquelle ausgesandten Stoßwellen werden über ein die Stoßwellen übertragendes Medium in den Körper eines Lebewesens eingekoppelt. Im Körper des Lebewesens sind die Stoßwellen beispielsweise auf einen Stein der Niere fokussiert, wo sie einen Teil ihrer Energie in Form von Druck- und Zugkräften zur Zerstörung des Konkrementes abgeben. Die Fokussierung der Stoßwelle kann beispielsweise durch eine akustische Linse erfolgen. Durch Verstellen der akustischen Linse in Richtung der akustischen Achse der Stoßwellen kann der Fokusbereich im Körper des Lebewesens längs der akustischen Achse der Stoßwellen verschoben werden. Der Fokusbereich kann also einer Ortsveränderung des Konkrements in Richtung der akustischen Achse der Stoßwellen nachgeführt werden.

Atembedingt oder bewegungsbedingt kann das Konkrement seine Lage aber auch quer zur akustischen Achse ändern. Zur Nachführung der akustischen Achse und damit des Fokusbereiches der Stoßwellen in dieser Richtung ist die Stoßwellenquelle an einer Halterung gehaltert, die eine allseitige Schwenkung des Stoßwellengenerators mit der Stoßwellenquelle und der akustischen Linse erlaubt.

Bedingt durch die zu bewegenden Massen ist diese Nachführung sehr träge, so daß der Fokusbereich nur sehr langsamen Ortsveränderungen des Konkrementes nachgeführt werden kann. Atmungsbedingte Ortsveränderungen des Konkrementes sind relativ schnell, ein Nachführen des Fokusbereiches ist praktisch nur schwer möglich. Verzichtet man auf diese Nachführung, so darf die Stoßwellenquelle zur Zerstörung des Konkrements nur aktiviert werden, wenn sich das Konkrement während einer Ortsveränderung im Fokusbereich der Stoßwellen befindet, wodurch die Behandlungszeit verlängert ist.

Aus der US-PS 3 913 061 ist eine Vorrichtung für ein bildgebendes Ultraschall-Diagnostikgerät bekannt, mit der die von einem Untersuchungsobjekt reflektierten Ultraschallsignale auf ein Linear-Array fokussiert und längs des Linear-Arrays abgelenkt werden können. Die Vorrichtung besitzt eine erste und eine zweite akustische Linse, mit der die reflektierten Ultraschallsignale auf das Linear-Array fokustiert werden. Im Bereich zwischen den akustischen Linsen sind zwei im Längsschnitt keilförmige Elemente um eine gemeinsame Achse drehbar gelagert und quer zur Richtung der Ultraschallsignale ausgerichtet. Die keilförmigen Elemente sind um 180° zueinander versetzt und von einer Antriebsvorrichtung mit unterschiedlicher Drehrichtung, aber jeweils gleicher Winkelgeschwindigkeit um die zentrale Achse drehbar. Bei Rotation der Elemente um die zentrale Achse werden die vom Objekt reflektierten und von der akustischen Linse auf das Linear-Array fokussierten Ultraschallsignale oszillierend entlang dem Linear-Array abgelenkt. Die akustischen Linsen und die keilförmigen Elemente sind in einem die Ultraschallsignale übertragenden Medium angeordnet.

Aufgabe der Erfindung ist es, eine Vorrichtung der eingangs genannten Art so auszubilden, daß die akustische Achse der Stoßwellen nahezu trägheitslos quer zur Ausbreitungsrichtung der Stoßwellen ausgelenkt werden kann.

Die Aufgabe wird erfindungsgemäß dadurch gelöst, daß in Ausbreitungsrichtung der Stoßwellen nach der Stoßwellenquelle eine akustische Ablenkvorrichtung angeordnet ist, die ein scheibenförmiges Element mit keilförmigem Längsschnitt aufweist und daß das scheibenförmige Element in einer Ebene etwa senkrecht zur Ausbreitungsrichtung der von der Stoßwellenquelle ausgesandten Stoßwellen verstellbar ist, wobei die Verstellung des scheibenförmigen Elementes eine Auslenkung der akustischen Achse der Stoßwelle bewirkt.

Vorteil ist, daß bedingt durch die geringe Masse des scheibenförmigen Elementes die akustische Achse der Stoßwelle praktisch trägheitslos einer schnellen (atembedingten) Ortsveränderung des Konkrements nachgeführt werden kann, daß damit die Stoßwellenquelle häufiger aktiviert werden kann, wodurch die Behandlungszeit reduziert ist.

Ein weiterer Vorteil ergibt sich dadurch, daß der Stoßwellengenerator an einem Bereich des Körpers angeordnet werden kann, an dem eine möglichst gute Stoßwellenankopplung möglich ist. Durch entsprechende Ausrichtung des scheibenförmigen Elementes kann die akustische Achse der Stoßwelle auf das Konkrement gerichtet werden.

Weitere Vorteile und Einzelheiten der Erfindung ergeben sich aus der nachfolgenden Beschreibung von Ausführungsbeispielen anhand der Zeichnungen in Verbindung mit den Unteransprüchen. Dabei zeigt:

FIG 1    ein erstes Ausführungsbeispiel einer Vorrichtung nach der Erfindung,

FIG 2    ein weiteres Ausführungsbeispiel einer Vorrichtung nach der Erfindung,

FIG 3    eine Ebene, in der der Fokusbereich der Stoßwelle abgelenkt werden kann und

FIG 4    das Prinzipschaltbild einer Ansteuerung einer Vorrichtung zum berührungslosen Zertrümmern eines Konkrementes mit einer akustischen Ablenkvorrichtung nach der Erfindung.

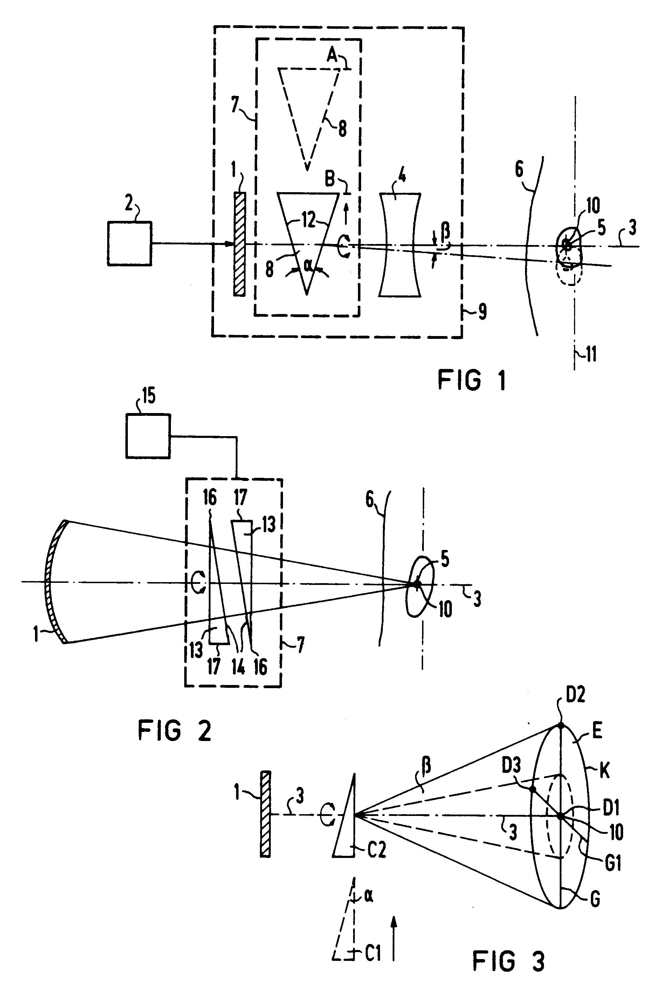

Die FIG 1 zeigt eine Stoßwellenquelle 1, die zum Erzeugen von Stoßwellen von einer Ansteuerschaltung 2 angesteuert wird. Eine Anordnung dieser Art ist zum Beispiel in der DE-OS 33 28 039 beschrieben. Die akustische Achse 3 der Stoßwellenquelle 1 ist in Richtung der ausgesandten Stoßwellen auf eine akustische Linse 4 zum Fokussieren der Stoßwellen und auf ein Konkrement 5 im Körper 6 eines Lebewesens gerichtet. Die FIG 1 zeigt, daß eine akustische Ablenkvorrichtung 7 zwischen der Stoßwellenquelle 1 und der akustischen Linse 4 angeordnet ist. Sie kann selbstverständlich auch in Stoßwellenrichtung nach der akustischen Linse 4 angeordnet sein. Ein scheibenförmiges Element 8 der akustischen Ablenkvorrichtung 7 besitzt einen keilförmigen Längsschnitt und ist in einer Ebene etwa senkrecht zur akustischen Achse 3 verstellbar.

Zu erwähnen ist, daß der Stoßwellengenerator 9, mit der Stoßwellenquelle 1, der akustischen Ablenkvorrichtung 7 und der akustischen Linse 4, sowie der Körper 6 des Lebewesens in einem die Stoßwellen übertragenden Medium, beispielsweise Wasser, angeordnet sein können. Es ist aber auch möglich, den Stoßwellengenerator 9 in einem nicht gezeigten Stoßwellenrohr anzuordnen, in dem sich das Stoßwellen übertragende Medium befindet und die von der Stoßwellenquelle 1 ausgesandten Stoßwellen über eine Ankoppelvorrichtung, beispielsweise einen Balg, der an der Stoßwellenaustrittsöffnung des Stoßwellenrohres befestigt ist, in den Körper 6 des Lebewesens einzukoppeln. Dabei steht die Stoßwellenaustrittsseite des Balges über ein Koppel-Gel in direktem Kontakt mit der Oberfläche des Körpers 6.

Wie aus der DE-OS 33 28 039 beispielsweise bekannt, wird der Stoßwellengenerator 9 bzw. der Körper 6 des Lebewesens so positioniert, daß der Fokusbereich 10 der Stoßwelle im Bereich des Konkrementes 5 angeordnet ist. Dabei kann das Element 8 der akustischen Ablenkvorrichtung 7 wirkungslos geschaltet sein. Dies wird in der FIG 1 durch die gestrichelt gezeichnete Position A des Elementes 8 angezeigt. Atmungsbedingt oder durch die dem Konkrement zugeführte Energie der Stoßwellen kann das Konkrement 5 eine Ortsveränderung beispielsweise in einer Richtung etwa senkrecht zur akustischen Achse 3 erfahren. Die Auslenkung der akustischen Achse 3 und damit die Nachführung des Fokusbereiches 10 erfolgt durch Verstellung des Elementes 8 der akustischen Ablenkvorrichtung 7. Die FIG 1 zeigt, daß sich das Konkrement 5 während der Atmung in Richtung der Körperlängsachse 11 verlagert hat. Zur Nachführung des Fokusbereiches 10 wird das Element 8 in die in der FIG 1 gezeigte Position B verstellt.

Der Betrag des Winkels $\beta$, um den die akustische Achse 3 ausgelenkt wird, ist dabei abhängig von der Schallgeschwindigkeit im Element 8, der Schallgeschwindigkeit in dem das Element 8 umgebenden Medium und vom Winkel $\alpha$, den die seitlichen Flächen 12 des Elementes 8 zueinander einnehmen.

Die FIG 3 zeigt beispielhaft eine Ebene E, in der der Fokusbereich 10 auslenkbar ist. Gleiche Elemente sind mit gleichen Bezugszeichen versehen. In der mit C1 gekennzeichneten Position des Elementes 8 erfolgt keine Auslenkung der akustischen Achse 3 der Stoßwellen und damit des Fokusbereiches 10. Die Position des Fokusbereiches 10 der Stoßwelle wird in der Ebene E mit D1 gekennzeichnet. Eine Verstellung des Elementes 8 in die Position C2 bewirkt die Auslenkung der akustischen Achse 3 um einen mit $\beta$ gekennzeichneten Winkel. Der Betrag des Winkel $\beta$ ist, wie vorher ausgeführt, abhängig vom Winkel $\alpha$ des Elementes 8, von der Schallgeschwindigkeit im Element 8 und von der Schallgeschwindigkeit im Medium, welches das Element 8 umgibt. Die Auslenkung des Fokusbereiches 10 wird in der Ebene E mit D2 gekennzeichnet. Eine Rotation des Elementes 8 in der Position C2 um die akustische Achse 3 um einen bestimmten Winkelbetrag bewirkt, daß beispielsweise der Fokusbereich 10 von der in der Ebene E mit D2 gekennzeichneten Position in die in der Ebene E mit D3 gekennzeichneten Position verstellt wird. Eine kontinuierliche Rotation des Elementes 8 um die akustische Achse 3 bewirkt, daß der Fokusbereich 10 der Stoßwelle auf der in der Ebene E mit K gekennzeichneten Kreisbahn rotiert.

Durch das Dazuschalten eines weiteren scheibenförmigen Elementes 8, welches in der FIG 3 nicht gezeigt ist, oder durch Verändern des Winkels $\alpha$ des Elementes 8 kann der Betrag des Winkels $\beta$, um den die akustische Achse 3 ausgelenkt wird, verändert werden.

Die FIG 1 zeigt lediglich ein Ausführungsbeispiel eines Elementes 8. So kann beispielsweise eine Fläche 12 senkrecht zur akustischen Achse 3 ausgerichtet sein. Es ist weiter möglich, daß eine Fläche 12 eine sich ändernde Steigung aufweist.

Die FIG 2 zeigt ein weiteres Ausführungsbeispiel einer akustischen Ablenkvorrichtung. Dabei ist die Stoßwellenquelle 1 so ausgeführt, daß die von ihr ausgesandten Stoßwellen auf das Konkrement 5 fokussiert sind. Im Ausführungsbeispiel besitzt die akustische Ablenkvorrichtung 7 zwei kreisscheibenförmige Elemente 13 mit keilförmigem Längsschnitt. Beide Elemente 13 sind um eine zentrale Längsachse drehbar gelagert. Die zentrale Längsachse ist in Richtung der akustischen Achse 3 und deckungsgleich zu dieser ausgerichtet. Die zur akustischen Achse 3 schräg verlaufenden Flächen 14 der Elemente 13 sind einander zugewandt.

Es ist ebenso möglich, die Flächen 14 in Richtung zur Stoßwellenquelle 1 oder in Richtung zum Körper 6, oder die Fläche 14 eines Elementes 13 in Richtung zur Stoßwellenquelle 1 und die Fläche 14 des anderen Elementes 13 in Richtung zum Körper 6 auszurichten.

Eine Rotation der Elemente 13 um die zentrale Längsachse wird durch eine Verstellvorrichtung 15 bewirkt. In Abhängigkeit von der Ansteuerung der Verstellvorrichtung 15 können Rotationen mit gleichem und unterschiedlichem Drehsinn und mit gleicher bzw. unterschiedlicher Winkelgeschwindigkeit durchgeführt werden. Es ist auch möglich, die Elemente 13 einzeln anzusteuern, so daß sie gegeneinander verstellbar sind.

Eine Rotation der Elemente 13 mit gleichem Drehsinn bewirkt, daß der Fokusbereich 10 der Stoßwelle auf einer Kreisbahn K rotiert (siehe FIG 3). Die größte Auslenkung, d.h. die längste Kreisbahn K beschreibt der Fokusbereich 10, wenn die im Längsschnitt der Elemente 13 gezeigten Keilspitzen 16 bzw. die Keilenden 17 deckungsgleich zueinander um die zentrale Längsachse rotieren. Keine Auslenkung des Fokusbereiches 10 wird erreicht, wenn eine Keilspitze 16 eines Elementes 13 und ein Keilende 17 des anderen Elementes 13 einander zugeordnet und die Elemente 13 gleich ausgebildet sind. Jede andere Zuordnung der Keilspitzen 16 bzw. der Keilenden 17 zueinander bewirkt eine Änderung des Durchmessers der Kreisbahn K, auf der der Fokusbereich 10 um die akustische Achse 3 rotiert.

Eine gegensinnige Rotation der Elemente 13 mit gleicher Winkelgeschwindigkeit um die zentrale Längsachse verstellt den Fokusbereich 10 längs einer Geraden G in der Ebene E senkrecht zur akustischen Achse 3 (siehe FIG 3). Eine Verstellung der Elemente 13 gegeneinander dreht die Gerade G in der Ebene E. Der Fokusbereich 10 wird dann, wie in der FIG 3 gezeigt, beispielsweise entlang der Geraden G1 in der Ebene E ausgelenkt.

Unterschiedliche Winkelgeschwindigkeiten der Elemente 13 lenken den Fokusbereich 10 in der Ebene E auf Bahnen, die von einer Geraden abweichen.

Die in der FIG 2 gezeigte fokussierende Stoßwellenquelle 1 kann selbstverständlich auch durch eine Stoßwellenquelle ersetzt werden, die ebene Stoßwellen aussendet. In einer vorteilhaften Ausführungsform ist dann die der Stoßwellenquelle 1 zugewandte Seite des in Stoßwellenrichtung ersten Elementes 13 und die der Stoßwellenquelle 1 abgewandte Seite des in Stoßwellenrichtung zweiten Elementes 13 beispielsweise konkav ausgebildet. Damit werden die von der Stoßwellenquelle 1 ausgesandten ebenen Stoßwellen auf das Konkrement 5 fokussiert. Es kann also auf eine zusätzliche fokussierende akustische Linse verzichtet werden.

Die FIG 1 und 2 zeigen lediglich Ausführungsbeispiele für eine akustische Ablenkvorrichtung 7. Wesentlich ist, daß mindestens ein Element 8, 13 der akustischen Ablenkvorrichtung 7 in einer Ebene etwa senkrecht zur akustischen Achse 3 der Stoßwellenquelle 1 verstellbar ist, so daß eine Auslenkung der akustischen Achse 3 bewirkt wird.

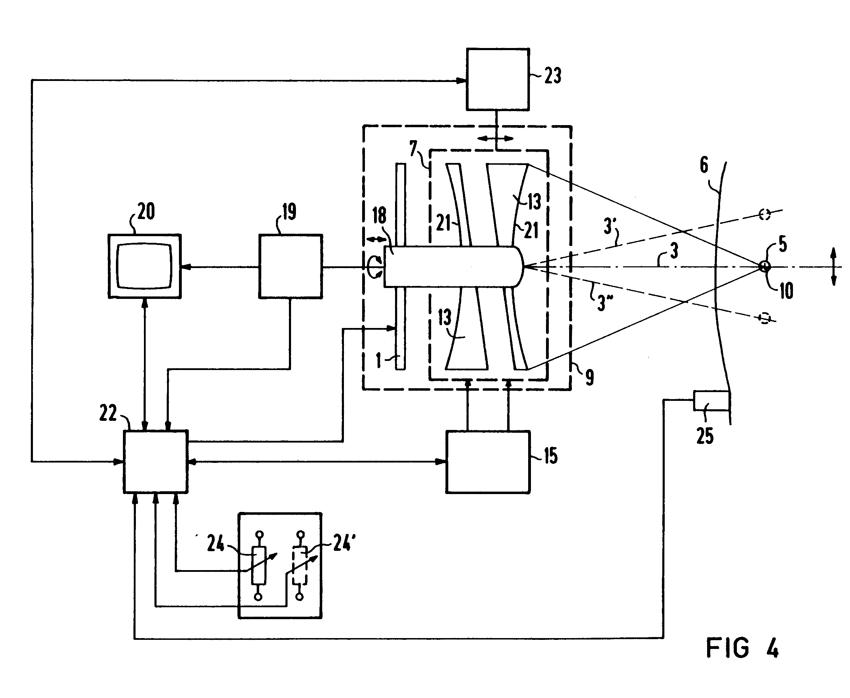

Die FIG 4 zeigt das Prinzipschaltbild einer Vorrichtung zum berührungslosen Zertrümmern eines Konkrementes mit einer Anordnung zum Orten und Darstellen des Konkrementes. Die Ortung und Darstellung des Konkrementes 5 erfolgt beispielsweise durch ein bekanntes Ultraschallgerät mit einer Ultraschalleinheit 18, mit einem Gerät 19 zur Verarbeitung der empfangenen Ultraschallsignale und mit einem Monitor 20 zum Darstellen des Untersuchungsbereiches. Die Ultraschalleinheit 18 ist, wie die FIG 4 zeigt, durch zentrale Öffnungen der Stoßwellenquelle 1 und der Elemente 13 geführt, in Richtung der akustischen Achse 3 verstellbar und um diese drehbar gelagert. Im Ausführungsbeispiel ist das Ultraschallgerät als B-Scan-Gerät ausgeführt, so daß durch dieses eine Ebene eines Untersuchungsbereiches auf dem Bildschirm des Monitors 20 darstellbar ist. Es ist zu erwähnen, daß die Elemente 13 der akustischen Ablenkvorrichtung 7 mit fokussierenden Flächen 21 versehen sind. Die Ortung des Konkrementes 5 kann in der aus der DE-OS 33 28 039 bekannten Weise erfolgen. Auf dem Bildschirm des Monitors 20 ist eine Markierung sichtbar, die den Fokusbereich der Stoßwelle anzeigt. Durch Verstellen der relativen Lage des Körpers 6 zum Stoßwellengenerator 9 bzw. durch Ortsveränderung des Stoßwellengenerators 9 relativ zum Körper 6 bzw. durch Verstellen der akustischen Ablenkvorrichtung 7 in Richtung der akustischen Achse 3 werden die Markierung und das Konkrement 5, welches ebenfalls auf dem Bildschirm des Monitors 20 sichtbar ist, zur Deckung gebracht. Dann befindet sich der Fokusbereich 10 der Stoßwelle im Körper 6 des Lebewesens im Bereich des Konkrementes 5. Beispielsweise atmungsbedingt kann das Konkrement 5 in der Ebene senkrecht zur akustischen Achse 3 verlagert werden. Diese Verlagerung ist auf dem Bildschirm des Monitors 20 sichtbar. Das Einstellen des Fokusbereiches 10 in Richtung der Ortsveränderung des Konkrementes 5 kann beispielsweise dadurch erfolgen, daß die Verstellvorrichtung 15 von einer Steuerschaltung 22 angesteuert wird, so daß die Markierung auf dem Monitor 20 zum Anzeigen des Fokusbereiches und damit der Fokusbereich 10 der Stoßwellen im Bereich der Ortsveränderung des Konkrementes 5 oszilliert. Dabei drehen die Elemente 13 mit unterschiedlichem Drehsinn und gleicher Winkelgeschwindigkeit um die zentrale Längsachse. Die Auslenkung der akustischen Achse 3 wird in der FIG 4 durch die gestrichelt gezeichneten Linien 3', 3'' dargestellt. Die Stoßwellenquelle 1 wird von der Steuerschaltung 22 aktiviert, wenn eine weitere steuerbare Markierung, die dem Konkrement 5 nachgeführt werden kann, deckungsgleich zur Markierung, die den Fokusbereich der Stoßwellen anzeigt, auf dem Bildschirm des Monitors 20 erscheint. Die Nachführung der steuerbaren Markierung kann durch ein Steuerglied 24, beispielsweise einen Trackball erfolgen, dessen Signale einer Markierungsgeberschaltung der Steuerschaltung 22 zugeführt werden. Zur Verkürzung der Behandlungszeit ist die Frequenz der Auslenkung des Fokusbereiches 10 höher als die Atemfrequenz, wenn z.B. Gallensteine behandelt werden sollen. Dann überschneiden sich der Fokusbereich 10 und das Konkrement 5 häufiger, so daß auch die Stoßwellenquelle 1 öfter aktiviert werden kann.

Es können auch Markierungen im Bereich der Ortsveränderung des Konkrementes 5 auf dem Bildschirm des Monitors 20 gesetzt werden. Die Stoßwellenquelle 1 wird aktiviert, wenn sich die Markierung zum Anzeigen des Fokusbereiches der Stoßwellen im Bereich dieser Markierungen befindet und ein Signal erfolgt, das mit der Ortsveränderung des Konkrementes 5 korreliert. Dabei kann es sich beispielsweise um ein von der Atmung abhängiges Signal handeln.

Durch Ansteuerung der Verstellvorrichtung 15 kann der Fokusbereich 10 einer Ortsveränderung des Konkrementes 5 aber auch manuell oder automatisch nachgeführt werden.

Eine manuelle Nachführung der Markierung zum Anzeigen des Fokusbereiches der Stoßwelle auf dem Bildschirm des Monitors 20 kann beispielsweise dadurch erfolgen, daß das Bedienpersonal diese Markierung über ein manuell betätigbares Steuerglied 24, beispielsweise einen Trackball, einer Ortsveränderung des Konkrementes 5 nachführt. Das Steuersignal des Steuergliedes 24 wirkt über die Steuerschaltung 22 auf die akustische Ablenkvorrichtung 7, dabei werden die Elemente 13 so gegeneinander verstellt, daß auch der Fokusbereich 10 der Stoßwelle im Körper 6 des Lebewesens der Ortsveränderung des Konkrementes 5 nachgeführt wird. Eine kontinuierliche Rotation der Elemente 13 ist hierzu nicht notwendig. Über ein weiteres Steuerglied 24' kann auch eine Verstellvorrichtung 23 zum Verstellen der akustischen Ablenkvorrichtung 7 längs der akustischen Achse 3 über die Steuerschaltung 22 angesteuert werden, um damit den Fokusbereich 10 der Stoßwelle bezüglich der Tiefe im Körper 6 des Lebewesens zu verstellen.

Vorteilhafterweise werden mindestens zwei B-Scan-Ebenen zum Darstellen des Konkrementes 5 auf dem Bildschirm des Monitors 20 angezeigt. Aufgrund dieser Darstellungen werden Ortsveränderungen des Konkrementes 5 sichtbar. Hierzu kann die Ultraschalleinheit 18 beispielsweise um einen bestimmten Winkelbetrag um die akustische Achse 3 gedreht werden. Es ist auch möglich, eine weitere, nicht gezeigte Ultraschalleinheit einzusetzen, wobei dann die B-Scan-Ebenen eine unterschiedliche Raumrichtung einnehmen können. Im jeweiligen Bild der B-Scan-Ebene ist eine Markierung zum Anzeigen des Fokusbereiches der Stoßwelle sichtbar. Zur Therapie des Konkrementes 5 werden durch Verstellen des Steuergliedes 24, 24' diese Markierungen mit dem Konkrement 5 auf dem Bildschirm des Monitors 20 zur Deckung gebracht, so daß die Stoßwellenquelle 1 angesteuert werden kann. Auf dem Bildschirm des Monitors 20 können selbstverständlich auch drei oder mehrere B-Scan-Ebenen dargestellt werden, so daß die Ortsveränderung des Konkrementes 5 in einem Volumenbereich dargestellt werden kann.

Es können beispielsweise auch drei B-Scan-Ebenen, die parallel zueinander sind, auf dem Bildschirm des Monitors 20 dargestellt werden. Das Konkrement 5 sollte dann in der mittleren B-Scan-Ebene auf dem Bildschirm des Monitors 20 dargestellt werden.

Eine Ortsveränderung und damit die Richtung der Ortsveränderung des Konkrementes 5 ist dadurch zu erkennen, daß das Konkrement in einer der weiteren B-Scan-Ebenen auf dem Bildschirm des Monitors 20 dargestellt wird. Die Nachführung des Fokusbereiches 10 der Stoßwellen kann durch entsprechende Ansteuerung der Ablenkvorrichtung 7 oder durch Verstellen des Stoßwellengenerators 9 erfolgen. Es ist vorteilhaft, wenn das Konkrement 5, nachdem der Fokusbereich 10 nachgeführt wurde, wieder in der mittleren B-Scan-Ebene dargestellt wird.

Eine automatische Nachführung des Fokusbereiches 10 der Stoßwelle kann beispielsweise dadurch erfolgen, daß auf dem Bildschirm des Monitors 20 und in jeder dargestellten B-Scan-Ebene Markierungen gesetzt werden, die die maximale Ortsveränderung des Konkrementes 5 anzeigen. Ein Rechner der Steuerschaltung 22 berechnet aus den Koordinaten, die durch die Markierungen gesetzt werden, und beispielsweise den atmungsabhängigen Signalen eines Gebers 25 die von der momentanen Atemlage abhängige, zu erwartende Ortsveränderung des Konkrementes 5. Der Fokusbereich 10 wird dann über die Steuerschaltung 22, die die Verstellvorrichtungen 23 und 15 ansteuert, automatisch nachgeführt.

Die Steuerschaltung 22 kann auch mit einer automatischen Bildauswertung ausgeführt sein, so daß durch diese die Koordinaten der Ortsveränderung des Konkrementes 5 festgelegt werden können und damit der Fokusbereich 10 der Stoßwelle ebenfalls automatisch nachgeführt werden kann.

Selbstverständlicherweise kann zur Ortung und Darstellung des Konkrementes auch eine Röntgenanlage eingesetzt werden.

**Patentansprüche**

1. Vorrichtung zum berührungslosen Zertrümmern eines Konkrementes (5) im Körper (6) eines Lebewesens mit einer Stoßwellenquelle (1) zum Erzeugen einer Stoßwelle in einem die Stoßwelle übertragenden Medium, wobei die Stoßwelle auf einen Bereich im Körper (6) des Lebewesens fokussiert ist, **dadurch gekennzeichnet,** daß in Ausbreitungsrichtung der Stoßwellen nach der Stoßwellenquelle (1) eine akustische Ablenkvorrichtung (7) angeordnet ist, die ein scheibenförmiges Element (8, 13) mit keilförmigem Längsschnitt aufweist und daß das scheibenförmige Element (8, 13) in einer Ebene etwa senkrecht zur Ausbreitungsrichtung der von der Stoßwellenquelle (1) ausgesandten Stoßwellen verstellbar ist, wobei die Verstellung des scheibenförmigen Elementes (8, 13) eine Auslenkung der akustischen Achse (3) der Stoßwelle bewirkt.

2. Vorrichtung nach Anspruch 1, **dadurch gekennzeichnet,** daß die akustische Ablenkvorrichtung (7) zwei kreisscheibenförmige Elemente (13) mit keilförmigem Längsschnitt aufweist, daß die kreisscheibenförmigen Elemente (13) um die zentrale Längsachse drehbar gelagert sind und daß eine Verstellvorrichtung (15) vorhanden ist zum Verstellen der kreisförmigen Elemente (13) um die zentrale Längsachse.

3. Vorrichtung nach Anspruch 2, **dadurch gekennzeichnet,** daß die kreisscheibenförmigen Elemente (13) gleich ausgebildet sind.

4. Vorrichtung nach einem der Ansprüche 2 und 3, **dadurch gekennzeichnet,** daß die kreisscheibenförmigen Elemente (13) individuell verstellbar sind.

5. Vorrichtung nach einem der Ansprüche 2 bis 4, **dadurch gekennzeichnet,** daß eine Verstellvorrichtung (15) vorhanden ist, durch die die Rotation der kreisscheibenförmigen Elemente (13) um die zentrale Längsachse steuerbar ist.

6. Vorrichtung nach Anspruch 5, **dadurch gekennzeichnet,** daß die Verstellvorrichtung (15) eine Rotation der kreisscheibenförmigen Elemente (13) mit unterschiedlichem Drehsinn um die zentrale Längsachse bewirkt.

7. Vorrichtung nach Anspruch 6, **dadurch gekennzeichnet,** daß die kreisscheibenförmigen Elemente (13) mit gleicher Winkelgeschwindigkeit um die zentrale Längsachse rotieren.

8. Vorrichtung nach einem der Ansprüche 1 bis 7 mit einer Anordnung zum Orten und Darstellen des Konkrements (5), wobei der Fokusbereich (10) der Stoßwelle auf das Konkrement (5) einstellbar und eine Ortsveränderung des Konkrementes (5) auf einem Monitor (20) der Anordnung zum Darstellen des Konkrementes (5) sichtbar ist, **dadurch gekennzeichnet,** daß der Fokusbereich (10) der Stoßwelle durch Ansteuerung der akustischen Ablenkvorrichtung (7) auf eine Bewegungsrichtung des Konkrementes (5) in einer Ebene etwa senkrecht zur Ausbreitungsrichtung der von der Stoßwellenquelle (1) ausgesandten Stoßwelle einstellbar ist.

9. Vorrichtung nach Anspruch 8, **dadurch gekennzeichnet,** daß die Ansteuerung der akustischen Ablenkvorrichtung (7) automatisch erfolgt, so daß der Fokusbereich (10) der Stoßwelle einer Ortsveränderung des Konkrementes (5) angepaßt ist.

10. Vorrichtung nach Anspruch 8, **dadurch gekennzeichnet,** daß die Ansteuerung der akustischen Ablenkvorrichtung (7) in Abhängigkeit von einer steuerbaren Markierung auf dem Monitor (20) erfolgt und daß die Markierung den Fokusbereich (10) der Stoßwelle anzeigt.

11. Vorrichtung nach Anspruch 8, 9 oder 10, **dadurch gekennzeichnet,** daß die Stoßwellenquelle (1) aktivierbar ist, wenn sich der Fokusbereich (10) der Stoßwelle im Bereich des Konkrementes (5) befindet.

12. Vorrichtung nach Anspruch 11, **dadurch gekennzeichnet,** daß die Stoßwellenquelle (1) aktivierbar ist, wenn sich das Konkrement (5) im Bereich einer Markierung auf dem Monitor (20) befindet, die in Bewegungsrichtung des Konkrementes (5) liegt.

## Claims

1. Device for the contactless disintegration of a calculus (5) in the body (6) of a living being, having a shock wave source (1) for generating a shock wave in a shock wave-transmitting medium, the shock wave being focussed on a region in the body (6) of the living being, characterised in that in the propagation direction of the shock waves after the shock wave source (1) an acoustic deflection device (7) is arranged which has a disc-shaped element (8, 13) with a wedge-shaped longitudinal section and in that the disc-shaped element (8, 13) can be adjusted in a plane approximately perpendicular to the propagation direction of the shock waves emitted by the shock wave source (1), the adjustment of the disc-shaped element (8, 13) causing a deviation of the acoustic axis (3) of the shock wave.

2. Device according to claim 1, characterised in that the acoustic deflection device (7) has two circular disc-shaped elements (13) with a wedge-shaped longitudinal section, in that the circular disc-shaped elements (13) are mounted rotatably about the central longitudinal axis and in that there is an adjustment device (15) for adjusting the circular elements (13) about the central longitudinal axis.

3. Device according to claim 2, characterised in that the circular disc-shaped elements (13) are identically shaped.

4. Device according to one of claims 2 and 3, characterised in that the circular disc-shaped elements (13) can be individually adjusted.

5. Device according to one of claims 2 to 4, characterised in that there is an adjustment device (15) by which the rotation of the circular disc-shaped elements (13) about the central longitudinal axis can be controlled.

6. Device according to claim 5, characterised in that the adjustment device (15) causes a rotation of the circular disc-shaped elements (13) in different rotational directions about the central longitudinal axis.

7. Device according to claim 6, characterised in that the circular disc-shaped elements (13) rotate at the same angular speed about the central longitudinal axis.

8. Device according to one of claims 1 to 7 having an arrangement for locating and displaying the calculus (5), the focus region (10) of the shock wave onto the calculus (5) being adjustable, and a change in position of the calculus being visible on a monitor (20) of the arrangement for displaying the calculus (5), characterised in that the focus region (10) of the shock wave can be adjusted by controlling the acoustic deflection device (7) to a direction of movement of the calculus (5) in a plane approximately perpendicular to the propagation direction of the shock wave emitted by the shock wave source (1).

9. Device according to claim 8, characterised in that control of the acoustic deflection device (7) occurs automatically so that the focus region (10) of the shock wave is adapted to a location change of the calculus (5).

10. Device according to claim 8, characterised in that control of the acoustic deflection device (7) occurs in dependence on a controllable mark on the monitor (20) and in that the mark indicates the focus region (10) of the shock wave.

11. Device acording to claim 8, 9 or 10, characterised in that the shock wave source (1) can be activated when the focus region (10) of the shock wave is in the region of the calculus (5).

12. Device according to claim 11, characterised in that the shock wave source (1) can be activated when the calculus (5) is in the region of a mark on the monitor (20) which lies in the direction of movement of the calculus (5).

## Revendications

1. Dispositif pour fragmenter à distance une concrétion (5) dans le corps (6) d'un être vivant, comportant une source d'ondes de choc (1) servant à produire une onde de choc dans un milieu transmettant l'onde de choc, l'onde de choc étant focalisée dans une zone située dans le corps (6) de l'être vivant, caractérisé par le fait qu'en aval de la source d'ondes de choc (1), et dans la direction de propagation des ondes de choc, est prévu un dispositif de déviation acoustique (7) qui comporte un élément en forme de disque (8,13) possédant une section longitudinale en forme de coin, et que l'élément en forme de disque (8,13) peut être déplacé dans un plan approximativement perpendiculaire à la direction de propagation des ondes de choc émises par la source d'ondes de choc (1), le déplacement de l'élément en forme de disque (8,13) provoquant une déviation de l'axe acoustique (3) de l'onde de choc.

2.  Dispositif suivant la revendication 1, caractérisé par le fait que le dispositif de déviation acoustique (7) comporte deux éléments en forme de disques circulaires (13) possédant une coupe longitudinale la forme d'un coin, que les éléments en forme de disques circulaires (13) sont montés de manière à pouvoir tourner autour de l'axe longitudinal central et qu'il est prévu un dispositif de réglage (15), qui sert à régler les éléments circulaires (13) autour de l'axe central longitudinal.

3.  Dispositif suivant la revendication 2, caractérisé par le fait que les éléments en forme de disques circulaires (13) sont identiques.

4.  Dispositif suivant l'une des revendications 2 et 3, caractérisé par le fait que les éléments en forme de disques circulaires (13) sont déplaçables individuellement.

5.  Dispositif suivant l'une des revendications 2 à 4, caractérisé par le fait qu'il est prévu un dispositif de réglage (15) qui permet de commander la rotation des éléments en forme de disques circulaires (13) autour de l'axe central longitudinal.

6.  Dispositif suivant la revendication 5, caractérisé par le fait que le dispositif de réglage (15) commande une rotation des éléments en forme de disques circulaires (13) avec un sens de rotation différent autour de l'axe longitudinal central.

7.  Dispositif suivant la revendication 6, caractérisé par le fait que les éléments en forme de disques circulaires (13) tournent avec la même vitesse angulaire autour de l'axe longitudinal central.

8.  Dispositif suivant l'une des revendications 1 à 7, comportant un dispositif pour localiser et représenter la concrétion (5), et dans lequel la zone focale (10) de l'onde de choc sur la concrétion (5) est réglable, et une modification de l'emplacement de la concrétion (5) est visible sur un moniteur (1) du dispositif servant à représenter la concrétion (5), caractérisé par le fait que la zone focale (10) de l'onde de choc est réglable, au moyen de la commande du dispositif de déviation acoustique (7), sur un dispositif de déplacement de la concrétion (5) dans un plan approximativement perpendiculaire à la direction de propagation de l'onde de choc émise par la source d'ondes de choc (1).

9.  Dispositif suivant la revendication 8, caractérisé par le fait que la commande du dispositif de déviation acoustique (7) s'effectue automatiquement de manière que la zone focale (10) de l'onde de choc soit adaptée à une variation locale de la concrétion (5).

10. Dispositif suivant la revendication 8, caractérisé par le fait que la commande du dispositif de déviation acoustique (7) s'effectue en fonction d'un marquage commandable sur le moniteur (20) et que le marquage indique la zone focale (10) de l'onde de choc.

11. Dispositif suivant la revendication 8, 9 ou 10, caractérisé par le fait que la source d'ondes de choc (1) peut être activée lorsque la zone focale (10) de l'onde de choc est située dans la zone de la concrétion (5).

12. Dispositif suivant la revendication 11, caractérisé par le fait que la source d'ondes de choc (1) peut être activée lorsque la concrétion (5) est située dans la zone d'un marquage sur le moniteur (20), qui se situe dans la direction de déplacement de la concrétion (5).

FIG 1

FIG 2

FIG 3

FIG 4

EP 0 355 176 B1